# EUROPEAN PATENT SPECIFICATION

(11) **EP 4 378 320 B1**
(45) Date of publication and mention of the grant of the patent: **30.09.2026**
(21) Application number: 22815400.1
(22) Date of filing: 29.07.2022
(51) Int. Cl.: A23K 20/121, A61K 31/341, A23K 50/10, A23K 50/20, A23K 50/30, A23K 50/40, A23K 50/75, A23K 50/80, A23K 50/60

(54) **USE OF FURANCARBOXYLIC ACIDS IN THE PREPARATION OF ADDITIVES FOR ANIMAL FEEDS**
VERWENDUNG VON FURANCARBONSÄUREN ZUR HERSTELLUNG VON FUTTERMITTELZUSATZSTOFFEN
UTILISATION D'ACIDES FURANCARBOXYLIQUES DANS LA PRÉPARATION D'ADDITIFS POUR ALIMENTS POUR ANIMAUX

(30) Priority: 30.07.2021 CN 202110871702
(43) Date of publication of application: 05.06.2024
(73) Proprietor: ANIPHA TECHNOLOGIES PTY LTD, Toorak Gardens, South Australia 5065 (AU)
(72) Inventor: PENG, Xianfeng, Guangzhou City, Guangdong 510663 (CN)
(74) Representative: Murgitroyd & Company
(86) International application number: PCT/CN2022/108803
(87) International publication number: WO 2022/253361

(56) References cited:
- CN-A- 101 053 369
- CN-A- 103 652 332
- CN-A- 104 161 187
- CN-A- 104 366 029
- CN-A- 108 514 050
- CN-A- 109 997 966
- CN-A- 113 768 046
- CN-B- 104 161 187
- US-A- 3 079 260
- SERAJUDDIN ET AL: "Salt formation to improve drug solubility", ADVANCED DRUG DELIVERY REVIEWS, ELSEVIER, AMSTERDAM , NL, vol. 59, no. 7, 24 August 2007 (2007-08-24), pages 603 - 616, XP022211982, ISSN: 0169-409X, [retrieved on 20241002], DOI: 10.1016/J.ADDR.2007.05.010

## Description

### FIELD OF THE INVENTION

The invention relates to the field of animal feed, in particular to the use of a compound of furancarboxylic acids, or a feed-acceptable salt thereof in the preparation of an additive for an animal feed or an animal feed for improving the growth performance of an animal.

### BACKGROUND OF THE INVENTION

Additives for feed refer to a small or trace amount of substances added during the feed processing, production and use, including nutritional additives for feed and non-nutritional additives for feed. Non-nutritional additives for feed refer to a small or trace amount of substances incorporated into the feed to ensure or improve the quality and utilization of the feed. The non-nutritional additives for feed that are commonly used in the art to efficiently and stably improve feed utilization and improve animal performance mainly comprise high-level copper, high-level zinc, feed antibiotics, chemically synthesized antibacterials, and the like. However, the long-term use of these substances in the breeding industry has serious side effects. In order to protect the health of animals and improve the production efficiency of the breeding industry, it is an urgent problem to be solved in the field to find effective, stable and safe new additives for feed.

### DESCRIPTION OF THE INVENTION

Accordingly, provided herein is the use of a compound of furancarboxylic acids or a feed-acceptable salt thereof in the preparation of an additive for an animal feed or of an animal feed for improving the growth performance of an animal.

In one aspect, provided herein is the use of a compound of Formula (I), or a feed-acceptable salt thereof in the preparation of an additive for an animal feed or of an animal feed for improving the growth performance of an animal: 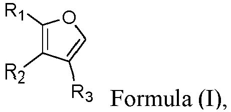
wherein each of R₁ and R₃ is H, and R₂ is -COOH; or R₁ is -COOH, and
each of R₂ and R₃ is H; or each of R₁ and R₃ is -COOH, and R₂ is H.

In some embodiments, the animal feed further comprises a feedstuff for an animal feed.

In some embodiments, the animal feed further comprises an additional additive for an animal feed.

In some embodiments, the animal feed further comprises a feedstuff and additional additive for an animal feed.

In some embodiments, the animal is livestock, poultry, an aquaculture animal, or a pet animal at various growth stages.

In some embodiments, the animal is livestock or poultry at the young stage

In some embodiments, for the livestock, such as pig, the compound of furancarboxylic acids, or a feed-acceptable salt thereof, or a feed composition comprising the same is added in an amount of 500 to 5000 ppm, preferably 2000 ppm to the animal feed.

In some embodiments, for the poultry, such as chicken, the compound of furancarboxylic acids, or a feed-acceptable salt thereof, or a feed composition comprising the same is added in an amount of 200 to 1000 ppm, preferably 1000 ppm to the animal feed.

In some embodiments, for the aquaculture animal, such as grass carp, the compound of furancarboxylic acids, or a feed-acceptable salt thereof, or a feed composition comprising the same is added in an amount of 100 to 600 ppm, preferably 500 ppm to the animal feed.

The technical effects in relation to the present invention are:
The compound of furancarboxylic acids, or a feed acceptable salt thereof, provided herein is capable of improving the growth performance of livestock, poultry, and aquaculture animals in the animal breeding test.

Any one of embodiments of any aspect provided herein can be combined with other embodiments as long as there is no contradiction between them. In addition, in any one of embodiments of any aspect provided herein, any one of technical features can be suitable for that technical feature in other embodiments, as long as there is no contradiction between them.

The foregoing described herein merely outlines certain aspects of the present invention, but is not limited to these aspects. The above aspects and other aspects will be described in more detail below.

### DESCRIPTION OF EXEMPLARY EMBODIMENTS

### Detailed Description of the Invention

Certain embodiments provided herein will now be described in detail, examples of which are illustrated by the accompanying structures and formulae. The disclosure intends to encompass all alternatives, modifications and equivalents, which are all within the scope of the disclosure In addition, certain technical features disclosed herein, which are, for clarity, described separately in multiple independent embodiments, can also be provided in combination in an individual embodiment or in any suitable subcombination.

### Definitions and/or Terminology of the Invention

"Feed-acceptable salt" as used herein, refers to a salt formed by a compound of furancarboxylic acids provided herein and an organic or inorganic base or an organic or inorganic acid that is non-toxic to animals. The term "feed-acceptable" refers to a substance or composition should be chemically or toxicologically suitable and relevant to the composition of the feed or to the farmed animal for consumption.

The "significant change" under a test condition for the study on influencing factors provided herein refers to "a change of more than 5% in the determination of the content of a test article."

"Animal" as used herein, refers to a human or a farmed animal that is unable to convert inorganics into organics, and can only use organics as food for life activities such as ingestion, digestion, absorption, respiration, circulation, excretion, sensation, movement, and reproduction. The term "farmed animal" comprises poultry, livestock, aquaculture animals, and other animals that are legally captured in captivity, including pet animals, such as cats and dogs. The term "livestock" refers to, for example, any one of pigs, cattle, horses, goats, sheep, deer and many useful rodents. The term "poultry" intends to include, for example, chickens, ducks, geese, quails, pigeons, and the like. The term "aquaculture animal" comprises, for example, fish, shrimp, turtles, soft-shelled turtles, and the like.

"Additive for a feed" or "additive for feed" as used herein, refers to a small or trace amount of substances added during feed processing, production and use, and is classified as a nutritional additive for a feed, and a general additive for a feed which is also called a non-nutritional additive for a feed.

"Feed" as used herein, refers to a product that is processed and produced by industrialization for animal consumption. The "feed" or "animal feed" comprises a feedstuff for an animal feed and one or more additives for an animal feed, unless otherwise stated.

"Composition" as used herein, refers to a set of compounds comprising one or more of the compounds provided herein as active ingredients.

"Comprising" or "comprises" as used herein, is an open expression, which not only includes those specified herein, but does not exclude other aspects.

### Use of Furancarboxylic Acids of the Invention

Provided herein is the use of a compound of furancarboxylic acids according to Formula (I), or a feed-acceptable salt thereof in the preparation of an additive for an animal feed for improving the growth performance of an animal: 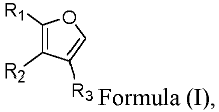
wherein each of R₁ and R₃ is H, and R₂ is -COOH; or R₁ is -COOH, and
each of R₂ and R₃ is H; or each of R₁ and R₃ is -COOH, and R₂ is H.

In some embodiments, the compound of furancarboxylic acids used in the preparation of an additive for an animal feed is preferably 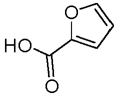 (chemical nomenclature: 2-Furancarboxylic acid).

In some embodiments, the compound of furancarboxylic acids used in the preparation of an additive for an animal feed is preferably 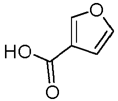 (chemical nomenclature: 3-Furancarboxylic acid).

In some embodiments, the compound of furancarboxylic acids used in the preparation of an additive for an animal feed is preferably 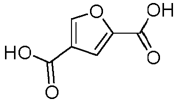 (chemical nomenclature: 2,4-Furandicarboxylic acid).

The compound of furancarboxylic acids or a feed-acceptable salt thereof is used in the preparation of a growth promoter for an animal in various growth stages, and the animal can be selected from livestock, poultry, an aquaculture animal, or a pet animal at various growth stages.

In further embodiments, the livestock include, but are not limited to, pigs, cattle, sheep, horses, rabbits, minks, or donkeys. The poultry include, but is not limited to, chickens, turkeys, ducks, geese, quails, or pigeons. The aquaculture animal includes, but is not limited to, fish, shrimp, turtles, crabs, soft-shelled turtles, bullfrogs, eels, or loaches. The pet animal includes, but is not limited to, dogs or cats of various subspecies.

In an embodiment, the compound of furancarboxylic acids or a feed-acceptable salt thereof is used in the preparation of an additive for a feed that can improve the growth growth performance of weaned piglets.

In another embodiment, the compound of furancarboxylic acids or a feed-acceptable salt thereof is used in the preparation of an additive for a feed that can improve the growth growth performance of medium pigs.

In another embodiment, the compound of furancarboxylic acids or a feed-acceptable salt thereof is used in the preparation of an additive for a feed that can improve the growth growth performance of chicks.

In another embodiment, the compound of furancarboxylic acids or a feed-acceptable salt thereof is used in the preparation of an additive for a feed that can improve the growth growth performance of fish.

Certain embodiments provided herein will now be described in detail, examples of which are illustrated by the accompanying structures and formulae. The disclosure intends to encompass all alternatives, modifications and equivalents, which are within the scope of the disclosure In addition, certain technical features disclosed herein, which are, for clarity, described separately in multiple independent embodiments, can also be provided in combination in an individual embodiment or in any suitable subcombination.

### EXAMPLES

### Example 1 Effects of furancarboxylic acids on growth performance of weaned piglets

A total of 120 65-day-old Duroc-landrace-yorkshire swine with similar body weight were randomly divided into 4 groups, each with 3 replicates, half male and female, 10 pigs in each group. The pigpen and utensils were disinfected before the test. During the period of test, pigs were kept in separate pens under the same feeding and management conditions. During the test, the test pigs were fed with food and water ad libitum, and were fed twice a day. The test groups are shown in Table 1, among which, group I was the control group, and was only fed with the basal diet; test groups II, III and IV were fed with the basal diet comprising 2000 ppm of a compound of furancarboxylic acids shown in Table 1, respectively. Each test group did not add other antioxidant components and growth promoters in the whole feeding process. The test lasted 28 days, and the growth performances of the test pigs, for example, the average daily feed intake (ADFI, g/d*pig), average daily weight gain (ADG, g/d*pig) and feed conversion ratio (FCR) of each pig were counted. The test results are expressed as "mean", as shown in Table 1. The results showed that the feed intake of the test pigs in the test groups fed with the basal diet comprising a compound of furancarboxylic acids was not significantly affected, but the average daily gain was significantly increased while the feed conversion ratio decreased by about 5.6%, compared with the control group.

**Table 1 Effects of furancarboxylic acids on growth performance of pigs**

| | **Test articles** | **Number of test pigs** | **ADFI** | **ADG** | **FCR** |
|---|---|---|---|---|---|
| group I | - | 10*3 | 1187 | 450 | 2.637 |
| group II | 2-Furancarboxylic acid | 10*3 | 1274 | 512 | 2.489 |
| group III | 3-Furancarboxylic acid | 10*3 | 1240 | 498 | 2.491 |
| group IV | 2,4-Furandicarboxylic acid | 10*3 | 1263 | 507 | 2.490 |

### Example 2 Study on the use of furancarboxylic acids in a feed for chicken

This experiment adopted a single-factor randomization design. 480 1-day-old broilers with an average body weight of 50 g were selected and randomly divided into 4 groups, with 6 replicates in each group, half male and female, 20 yellow-feathered broilers per replicate. The chicken house and utensils were disinfected before the test. During the period of test, broilers were kept in cages under the same feeding and management conditions. The basal diet is mainly based on com-soybean meal, and other antioxidants and growth promoters are not added during the whole feeding. The test groups are shown in Table 2, among which, group I was the control group, and was only fed with the basal diet; test groups II, III and IV were fed with the basal diet comprising 1000 ppm of a compound of furancarboxylic acids shown in Table 2, respectively. The test lasted 20 days, and the test broilers were fed with food and water ad libitum, and were fed twice a day. Test broilers in each replicate were weighed at 21 days of age (stop feeding for 12 hours and keep water). The feed consumption of the test broilers was counted, and the average daily feed intake (ADFI, g/d*broiler), average daily weight gain (ADG, g/d*broiler) and feed conversion ratio (FCR) of each test broiler were calculated.

The test results are expressed as "mean", as shown in Table 2. The results showed that the effect of the feed intake of the test chickens in each test group was not significant, but the average daily gain of the test chickens was significantly increased and the feed conversion ratio was significantly decreased, compared with the control group.

**Table 2 Effects of furancarboxylic acids on growth performance of broilers**

| | **Test articles** | **Number of test broilers** | **ADFI** | **ADG** | **FCR** |
|---|---|---|---|---|---|
| group I | - | 20*6 | 32.6 | 14.8 | 2.20 |
| group II | 2-Furancarboxylic acid | 20*6 | 35.2 | 16.9 | 2.08 |
| group III | 3-Furancarboxylic acid | 20*6 | 34.0 | 16.4 | 2.07 |
| group IV | 2,4-Furandicarboxylic acid | 20*6 | 36.5 | 17.4 | 2.10 |

### Example 3 Study on the use of furancarboxylic acids in a feed for fish

### (1) Experimental material

Test fish: The test fish used were the healthy and lively grass carp fingerlings of the same year and were reared in a large cage (4 x 2 x 1.5 m³) for 4 weeks before being used for formal breeding tests. The experimental system was a small floating cage (with size of 1.1 x 1.1 x 1.1 m³); each small cage is equipped with a bubbling head, and bubbling 24 hours a day. The small cages and the temporary cages were placed in a test facility, a 3500 m² pond, the pond depth was about 1.5 m, and the pond water was fully aerated bottom water. During the test, 240 grass carps that had been starved for 1 day were randomly divided into 3 groups, each group was provided with 4 replicates, and 20 fish were placed in each replicate. Each replicate was weighed and placed into 12 cages, respectively fed with a test feed supplemented with a compound of furancarboxylic acids.

Test feed: The test feed was prepared according to the formula in Table 3, and 500 ppm of a compound of furancarboxylic acids were added to different test groups according to Table 4. The feedstuff used was ultrafinely grinded and then passed through the Jiangsu Muyang extruder to make a floating extruded feed with a particle size of 3 mm. The mold release temperature was 130 °C, and 3% soybean oil was sprayed externally through the oil injection equipment, and sealed in a cool place for use.

**Table 3 Feed formula for grass carp and chemical ingredients (wt %)**

| **Ingredients** | **Content (%)** | **Ingredients** | **Content (%)** |
|---|---|---|---|
| fish meal | 9.0 | soybean oil | 3.0 |
| hog casing powder | 3.0 | phospholipid rapeseed meal | 9.0 |
| soybean meal | 12.0 | gluten | 4.0 |
| rapeseed meal | 12.0 | blood cell powder | 2.0 |
| MSG protein | 3.0 | Vc-Phosphate | 0.1 |
| wheat middlings | 12.6 | calcium dihydrogen phosphate | 1.8 |
| flour | 17.0 | choline chloride | 0.2 |
| bentonite | 0.70 | multivitamins | 0.1 |
| rice bran | 10.0 | micro-mineral premix | 0.5 |

**Table 4 Growth-promoting test groups of furancarboxylic acids**

| **Groups** | **Test articles** | **Amount (ppm)** |
|---|---|---|
| control | - | - |
| group I | 2-Furancarboxylic acid | 500 |
| group II | 3-Furancarboxylic acid | 500 |

### (2) Experimental method

Procedures: The experiment adopts artificial food restriction feeding, the feeding amount is adjusted once a week, the feeding level (based on the initial body weight) of each group is exactly the same, feeding twice a day (at 7:30 am and 15:00 pm), and the total feeding amount is 580 g per replicate. The test lasted 8 weeks. The water quality was monitored regularly during the test. The water temperature was 26.88 ± 3.08 °C, with DO > 5.0 mg O L⁻¹, pH 7.8, ammonia nitrogen < 0.50 mg N L⁻¹, and nitrite nitrogen < 0.05 mg N L⁻¹ during the whole breeding process.

Parameter statistics: During the test, the fish in each cage were weighed as a whole 1 day of stopping feeding, and their weight gain (WG, %), feed conversion rate (FCR) and survival rate (SR, %) were calculated as follows: 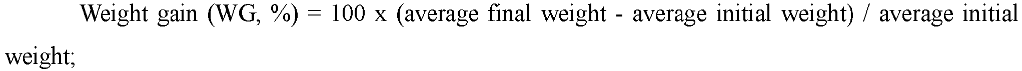 Weight gain (WG, %) = 100 x (average final weight - average initial weight) / average initial weight; $Feed conversion rate \left(FCR\right) = feed intake / weight gain of fish ;$ 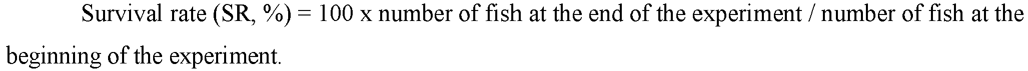 Survival rate (SR, %) = 100 x number of fish at the end of the experiment / number of fish at the beginning of the experiment.

### (3) Experimental results

The results of the effect of furancarboxylic acids on the growth performance of grass carps are shown in Table 5. The results showed that the test groups added with a compound of furancarboxylic acids were better than the control group in terms of weight gain and feed conversion rate, and had obvious growth-promoting effects. In addition, the survival rate and anti-hypoxia ability of grass carp were significantly improved in test groups.

**Table 5 Test results of the use of furancarboxylic acids in a feed for aquaculture animals**

| | **Average initial weight (g)** | **Average final weight (g)** | **Weight gain (%)** | **(FCR)** | **SR (%)** |
|---|---|---|---|---|---|
| control | 251.25 | 574.64 | 133.15 | 1.56 | 55.00 |
| group I | 249.81 | 586.87 | 134.96 | 1.48 | 82.50 |
| group II | 246.56 | 589.33 | 138.98 | 1.48 | 83.75 |

The foregoing examples merely represent several embodiments of the invention, and there are other embodiments to perform the invention. Correspondingly, the embodiments of the invention will be illustrated as examples, but should not be regarded as limiting the scope of the present invention, as defined by the appended claims.

## Claims

1. Use of a compound of Formula (I), or a feed-acceptable salt thereof in the preparation of an additive for an animal feed for improving the growth performance of an animal: 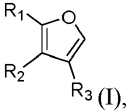 wherein each of R₁ and R₃ is H, and R₂ is -COOH; or R₁ is -COOH, and each of R₂ and
R₃ is H; or each of R₁ and R₃ is -COOH, and R₂ is H.

2. The use of claim 1, wherein the additive for the animal feed is an additive for a feed for livestock, poultry, aquaculture animals, or pet animals at various growth stages.

3. The use of claim 1, wherein the additive for the animal feed is a growth promoter.

4. Use of a compound of Formula (I), or a feed-acceptable salt thereof in the preparation of an animal feed for improving the growth performance of an animal: 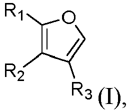 wherein each of R₁ and R₃ is H, and R₂ is -COOH; or R₁ is -COOH, and each of R₂ and R₃ is H; or each of R₁ and R₃ is -COOH, and R₂ is H.

5. The use of claim 4, wherein the animal feed is a feed for livestock, poultry, aquaculture animals, or pet animals at various growth stages.

6. The use of claim 4, wherein the animal feed further comprises a feedstuff for an animal feed.

7. The use of claim 4, wherein the animal feed further comprises an additional additive for an animal feed.

8. The use of claim 7, wherein the additional additive for the animal feed is a nutritional additive for a feed, a non-nutritional additive for a feed, or a pharmaceutical additive for a feed.

## Patentansprüche

1. Verwendung einer Verbindung der Formel (I) oder eines futtermittelverträglichen Salzes davon bei der Herstellung eines Zusatzstoffes für ein Futtermittel zur Verbesserung der Wachstumsleistung eines Tieres: 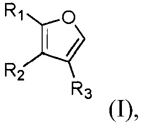 wobei R₁ und R₃ jeweils H sind und R₂ -COOH ist; oder R₁ -COOH ist und R₂ sowie R₃ jeweils H sind; oder R₁ und R₃ jeweils -COOH sind und R₂ H ist.

2. Verwendung nach Anspruch 1, wobei der Zusatzstoff für das Futtermittel ein Zusatzstoff für ein Futtermittel für Nutztiere, Geflügel, Tiere in Aquakultur oder Heimtiere in verschiedenen Wachstumsstadien ist.

3. Verwendung nach Anspruch 1, wobei der Zusatzstoff für das Futtermittel ein Wachstumsförderer ist.

4. Verwendung einer Verbindung der Formel (I) oder eines futtermittelverträglichen Salzes davon bei der Herstellung eines Futtermittels zur Verbesserung der Wachstumsleistung eines Tieres: 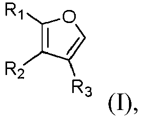 wobei R₁ und R₃ jeweils H sind und R₂ -COOH ist; oder R₁ -COOH ist und R₂ sowie R₃ jeweils H sind; oder R₁ und R₃ jeweils -COOH sind und R₂ H ist.

5. Verwendung nach Anspruch 4, wobei das Futtermittel ein Futtermittel für Nutztiere, Geflügel, Tiere in Aquakultur oder Heimtiere in verschiedenen Wachstumsstadien ist.

6. Verwendung nach Anspruch 4, wobei das Futtermittel ferner ein Futtermittel-Ausgangserzeugnis umfasst.

7. Verwendung nach Anspruch 4, wobei das Futtermittel ferner einen weiteren Zusatzstoff für ein Futtermittel umfasst.

8. Verwendung nach Anspruch 7, wobei der weitere Zusatzstoff für das Futtermittel ein ernährungsphysiologischer Zusatzstoff für ein Futtermittel, ein nicht ernährungsphysiologischer Zusatzstoff für ein Futtermittel oder ein pharmazeutischer Zusatzstoff für ein Futtermittel ist.

## Revendications

1. Utilisation d'un composé de formule (1), ou d'un sel acceptable pour l'alimentation animale de celui-ci, dans la préparation d'un additif pour un aliment pour animaux destiné à améliorer les performances de croissance d'un animal : 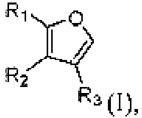 dans laquelle chacun de R₁ et R₃ est H, et R₂ est -COOH ; ou R₁ est -COOH, et chacun de R₂ et R₃ est H ; ou chacun de R₁ et R₃ est -COOH, et R₂ est H.

2. Utilisation selon la revendication 1, dans laquelle l'additif pour l'aliment pour animaux est un additif pour un aliment destiné aux animaux d'élevage, à la volaille, aux animaux d'aquaculture ou aux animaux de compagnie à divers stades de croissance.

3. Utilisation selon la revendication 1, dans laquelle l'additif pour l'aliment pour animaux est un promoteur de croissance.

4. Utilisation d'un composé de formule (I), ou d'un sel acceptable pour l'alimentation animale de celui-ci, dans la préparation d'un aliment pour animaux destiné à améliorer les performances de croissance d'un animal : 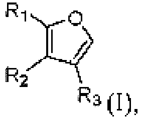 dans laquelle chacun de R₁ et R₃ est H, et R₂ est -COOH ; ou R₁ est -COOH, et chacun de R₂ et R₃ est H ; ou chacun de R₁ et R₃ est -COOH, et R₂ est H.

5. Utilisation selon la revendication 4, dans laquelle l'aliment pour animaux est un aliment destiné aux animaux d'élevage, à la volaille, aux animaux d'aquaculture ou aux animaux de compagnie à divers stades de croissance.

6. Utilisation selon la revendication 4, dans laquelle l'aliment pour animaux comprend en outre une matière première pour un aliment pour animaux.

7. Utilisation selon la revendication 4, dans laquelle l'aliment pour animaux comprend en outre un additif supplémentaire pour un aliment pour animaux.

8. Utilisation selon la revendication 7, dans laquelle l'additif supplémentaire pour l'aliment pour animaux est un additif nutritionnel pour un aliment, un additif non-nutritionnel pour un aliment ou un additif pharmaceutique pour un aliment.
